# EUROPEAN PATENT APPLICATION

(11) **EP 1 595 447 A1**
(43) Date of publication of application: **16.11.2005**
(21) Application number: 03703094.7
(22) Date of filing: 30.01.2003
(51) Int. Cl.: A01K 67/027, C12N 15/09, C12N 5/10

(54) **TRANSGENIC ANIMAL HAVING FATTY ACID DESATURASE AND METHOD OF PRODUCING THE SAME**

(71) Applicant: Kinki University, Higashiosaka-shi Osaka 577-8502 (JP); Japan Society for the Promotion of Science, Tokyo 102-8471 (JP)
(72) Inventor: SAEKI, Kazuhiro, Naga-gun, Wakayama 649-6255 (JP); MATSUMOTO, Kazuya, Naga-gun, Wakayama 649-6255 (JP); KINOSHITA, Mikio, Oibihiro-shi, Hokkaido 080-0834 (JP); SUZUKI, Iwane; Kenei Ohnishi Jutaku 304,, Okazaki-shi, Aichi 444-0812 (JP); TAGUCHI, Yoshitomo, Wakayama-shi, Wakayama 649-6331 (JP); MIKAMI, Koji; Central-Nishiki 402, 139,, Okazaki-shi, Aichi 444-0868 (JP); UEDA, Masatsugu, Kawagoe-shi, Saitama 350-1151 (JP); HOSOI, Yoshihiko, Sakai-shi, Osaka 590-0144 (JP); MURATA, Norio, Okazaki-shi, Aichi 444-0817 (JP); IRITANI, Akira, Kyoto-shi, Kyoto 606-0026 (JP); KANO, Kouichiro, Fujisawa-shi, Kanagawa 252-0813 (JP)
(74) Representative: Wibbelmann, Jobst
(86) International application number: PCT/JP2003/000930
(87) International publication number: WO 2004/066724

(57) **Abstract**

To provide meat that is beneficial to human health, for the purpose to produce transgenic animals in which the content of unsaturated fatty acids increase is increased, transgenic animals **characterized by** increased content of unsaturated fatty acids that are beneficial to human health is provided by the present invention. Furthermore, the present invention also provides a method to enhance levels of unsaturated fatty acid in animals.

SEQUENCE LISTING
<110> School juridical organization Kinki University, Japan Society
for the Promotion of Science
<120> A transgenic animal carrying fatty acid desaturases and a
method for production of the animal
<130> GF 2002-461
<150> JP 2001-387638
<151> 2001-12-20
<160> 1
<170> PatentIn version 3.1
<210> 1
<211> 1640
<212> DNA
<213> Spinatica oleracea L.
<400> 1
aagcttgtcg aaattcgcga gccggcgcac acttcggcct ctccttctcc ttcctcaaac 60
caaaaaaaaa gtctctctgt ttttatttaa ttttttaaaa ttaattaatt cagagagcaa 120
aaaaataaat cacagatttc gaggttgaag aagttcgcaa tttttgatgt accacttcga 180
gcggctggaa caatgggtgc aggtgggaga tctattcctc catcggcgag aaaggagaaa 240
tctgatgcat tgaacagagt accatacgaa aaaccaccat tcacactagg gcagataaaa 300
aaagccatcc cacctcattg cttcaaacgc tctgtgctac gctctttctc ctatgtggtt 360
tatgatttca ccattgcgtt cctcctctac tatgttgcta ctaactacat ccacctcctt 420
ccaaagcctt tcaactactt ggcttggcct gtgtatggat ttgtccaagg ctgtgttctt 480
accggtgttt gggttatagc ccatgaatgt ggccaccatg ccttcagtga ttaccagtgg 540
ctcgatgaca ctgttggctt agtcctccac tcgttccttc ttgtgccata tttctcatgg 600
aaatacagtc acaggcgcca tcactcaaac actggttcaa tggagaaaga tgaagttttt 660
gtaccaaaaa gaaaggaaaa tatgtcatgg ttttccaagt atcttagcaa cccacctgga 720
cgaatcctga cccttgttgt gacgctaacc cttggctggc ctttgtatct tctgtttaat 780
gtatcgggta ggaaatatga gcgttttgct tgccattatg acccatcctc tccaatctac 840
tcggaccgtg agaggcttca aatattcatt tctgatgttg ggatttcgtt agtggctttt 900
gggctttatc accttgcagc tgccaaagga atttcatggg tgttgtgtgt atatgggggt 960
ccattgcttg ttgtgaacgg atttcttgtc ctgattacct tccttcagca cacacaccct 020
tcattgcccc attacgatac atctgaatgg gattggttga gaggtgcatt ggctaccgtg 1080

gaccgagact acgggatttt gaacaaggtg ttccacaaca ttactgatac tcacgtggct 1140

caccatctca tctcgaccat gccccattat catgctatgg aggcaaccaa ggctataaag 1200

ccaatattgg gcaaatatta tcggttggat tcaactccag tattcaaggc aatgtggagg 1260

gaggccaaag aatgtatgta tgtggaggct gatgaagacg accagaacaa aagtgtgctc 1320

tggtacagaa acaagcttgg aaaatcctgg tttctccttt agggattagc cttctcttat 1380

cctttaaaac cccctcccta ctagatttat aggttggttg tgtgtttgtc atgttttgta 1440

tttttgaacc gcggatttct ttaactacca ttccagtaat gttgatcatt gtgatagagc 1500

gagaatcact aggcgagttg ggggagttgt ttgttattgt tgtttttgca ataatctgta 1560

catccttgct gtgcccagat ttcttggcat atctacggat gggacacaat tatttgtgaa 1620

tttttgcggc ccgcgaattc 1640

## Description

### BACKGROUND OF THE INVENTION

### 1. Technical Field of the Invention

The present invention relates to transgenic animals and animal cells introduced with (a) gene(s) for (a) fatty acid desaturase(s) (an enzyme catalyzing the desaturation of fatty acid), and this invention also relates to a method for increasing the content of unsaturated fatty acids in animals.

### 2. Description of the Related Art

Fats are not only a very important energy source, but also are a source of essential fatty acids (linoleic acid (18:2n-6), α-linolenic acid (18:3n-3) and arachidonic acid (18:4n-3)) for normal growth and maintaining function of mammals. In addition to the above, Crawford et al. (Am. J. Clin. Nutr., 31, 2181-2185, 1978) included n-3 polyunsaturated fatty acids(PUFAs) into the essential fatty acids, for it was revealed that eicosapentaenoic acid (EPA, 20:5n-3) and docosahexaenoic acid (DHA, 22:6n-3) were required for learning ability and development of visual perception. Based on etiological studies, it has been indicated that intake of sufficient amounts of these PUFAs can reduce the risk of coronary heart disease and thrombotic diseases (Dyerberg and Bang, Lancet, 433-435 (1979); Harris, J. Lipid Res., 30, 785-807 (1989); and Kinsella et al., Am. J. Clin. Nutr., 52, 1-28 (1990)).

Most of animals including human do not have enzymes for synthesis of those PUFAs (Innis, Prog. Lipid Res., 30, 39-103 (1991)). More specifically, most animals can not convert oleic acid (18:1) to linoleic acid (18:2n-6) nor linoleic acid to α-linolenic acid (18:3n-3) in situ. On the other hand, because Δ12 and Δ15 fatty acid desaturases (FAD) exist in plants, they can synthesize these PUFAs by themselves. Thus, vegetable oils contain a large quantities of PUFAs EPA and DHA, as well as linoleic acid and α-linolenic acid. The Δ12 fatty acid desaturase used herein means an enzyme catalyzing the reaction of forming a double bond at the 12th position numbered from the carboxyl group at the terminal of the carbon chain of a fatty acid. In the same manner, the Δ15 fatty acid desaturase used herein means an enzyme catalyzing the reaction of forming a double bond at the 15th position numbered from the carboxyl group at the terminal of the carbon chain of a fatty acid.

Recently the dining habit of North America has spread in Japan. With this tendency, excess intake of animal fat has brought about the increased risk of coronary heart diseases and thrombotic diseases in Japan. According to the US Guidance for the Dietary Management of Hypercholesterolemia (The Expert Panel, Arch. Intern. Med., 148, 36 (1988)), it is recommended to keep the calories ascribed to total fat and saturated fatty acids, and to mono- and poly-unsaturated fatty acids at 30% or lower and 7% or lower, and at 10-15% or higher and 10% or higher of the total ingested calorie, respectively. Namely, the Guidance recommended to reduce intake of animal fat and to increase intake of vegetable fat.

### SUMMARY OF THE INVENTION

As far as based on current technology, increased intake of polyunsaturated fatty acids (PUFAs) would be achieved only by reducing intake of animal meat and increasing intake of fish meat and vegetable oil. In the animal body, the ingested linoleic acid can be converted to arachidonic acid (20:4n-3/n-6) and α-linolenic acid can be converted to EPA and then to DHA in situ. Then it was attempted to introduce genes for Δ12 and/or Δ15 fatty acid desaturases into an animal particularly livestock for meat. And if biosynthesis of essential fatty acids, particularly linoleic acid and/or α-linolenic acid which can be naturally bio-synthesized only by vegetables, would be achieved in animals using genetic engineering technology, animal meat containing a large amount of PUFAs would be produced. This would contribute to production of animal meat that would be beneficial to human health without need of consideration of the amount of meat ingestion.

Chemical formula 1 below represents the structure of palmitic acid (16 carbons with no unsaturated bond, 16:0). Chemical formula 2 represents the structure of stearic acid (18 carbons with no unsaturated bond, 18:0). Oleic acid is a fatty acid which has a double bond between the 9th and 10th carbon atoms numbered from the carboxyl group terminal of the carbon chain consisting of 18 carbons, and chemical formula 3 represents the structure of oleic acid (18 carbons with one unsaturated bond, 18:1). Oleic acid is converted, via the action of Δ12 fatty acid desaturase, into linolenic acid which contains an additional double bond formed at the 12th position. Chemical formula 4 represents the structure of linolenic acid (18 carbons with two unsaturated bonds, 18:2). Linolenic acid is converted, via the action of Δ15 fatty acid desaturase, into α-linolenic acid which contains an additional double bond formed at the 15th position. Chemical formula 5 represents the structure of α-linolenic acid (18 carbons with three unsaturated bonds, 18:3).
Chemical formula 1
COOH-CH₂-CH₂-CH₂-CH₂-CH₂-CH₂-CH₂-CH₂-CH₂-CH₂-CH₂-CH₂-CH₂-CH₂-CH₃
Chemical formula 2
COOH-CH₂-CH₂-CH₂-CH₂-CH₂-CH₂-CH₂-CH₂-CH₂-CH₂-CH₂-CH₂-CH₂-CH₂-CH₂-CH₂-CH₃
Chemical formula 3
COOH-CH₂-CH₂-CH₂-CH₂-CH₂-CH₂-CH₂-CH=CH-CH₂-CH₂-CH₂-CH₂-CH₂-CH₂-CH₂-CH₃
Chemical formula 4
COOH-CH₂-CH₂-CH₂-CH₂-CH₂-CH₂-CH₂-CH=CH-CH₂-CH=CH-CH₂-CH₂-CH₂-
CH₂-CH₃
Chemical formula 5
COOH-CH₂-CH₂-CH₂-CH₂-CH₂-CH₂-CH₂-CH=CH-CH₂-CH=CH-CH₂-CH=CH-CH₂-CH₃

The first aspect of the present invention is to provide a transgenic animal in which a gene for a fatty acid desaturase is introduced into the animal to transform the animal to allow the gene for a fatty acid desaturase to be expressed in the animal, thereby the content of unsaturated fatty acid is increased in the animal.

The second aspect of the present invention is to provide a transgenic animal cell in which a gene for a fatty acid desaturase is introduced into the animal cell to transform the animal cell to allow the gene for a fatty acid desaturase to be expressed in the animal cell, thereby the content of unsaturated fatty acid is increased in the animal cell.

The third aspect of the present invention is to provide method for increasing the content of unsaturated fatty acids in an animal, the method comprising the steps of introducing a gene for fatty acid desaturase in an animal, and transforming the animal to allow the gene for fatty acid desaturase to be expressed in the animal.

The fourth aspect of the present invention is to provide a method for increasing the content of unsaturated fatty acids in an animal, the method comprising the steps of constructing a vector containing a gene for a fatty acid desaturase with a transcriptional control region that enables the expression of the gene for fatty acid desaturase, transforming animals by introduction of the vector into early embryos, somatic cells or embryonic stem cells (ES cells) of the animals, and allowing the gene for a fatty acid desaturase to be expressed in the animals that were developed from the transformed early embryos, somatic cells or embryonic stem cells.

The present invention will be described in detail below. However, description of the preferred embodiments and examples given below is not intended in any way to limit or restrict the effective scope of the present invention.

### BRIEF DESCRIPTION OF THE DRAWINGS

Fig. 1 presents photographs of RT-PCR analysis and Northern blotting analysis indicating the expression of spinach-derived fad2 gene in the transgenic mice.

Fig. 2 presents a photograph of Western blotting analysis indicating the expression of spinach-derived FAD2 in the brown adipose tissue of a transgenic mouse.

Fig. 3 is a graph indicating the composition of fatty acids in the total lipids of the brown adipose tissue of wild-type mice and B1 transgenic mice fed a normal diet or a high-oleic-acid diet.

Fig. 4 presents photographs of Southern blotting analysis indicating integration of the introduced gene into chromosome of a transgenic pig and its piglets.

Fig. 5 presents photographs of RT-PCR analysis indicating the expression of fad2 mRNA in the white adipose tissue of transgenic pigs (B3, B12 and B19).

Fig. 6 presents photographs of Northern blotting analysis indicating the expression of fad2 mRNA in a transgenic pig of B-12 line.

Fig. 7 is a graph indicating the composition of fatty acids in the total lipids of the white adipose tissue obtained from transgenic pigs carrying aP2/fad2 (B-12 breed line) fed a normal diet.

### DESCRIPTION OF THE PREFERRED EMBODIMENTS

The present inventors isolated a gene for a fatty acid desaturase bound to the membrane of endoplasmic reticulum of a plant, injected it into fertilized eggs of mice or pigs to achieve stable integration into their chromosomes, and developed the eggs to live animals. Detailed studies on such animals indicated that the introduction of the gene did not affect in any way to the normal growth and the function of these animals. The introduced gene for a fatty acid desaturase could be expressed and exert its function, thereby the composition of fatty acids were altered and the content of PUFAs would be increased in the transgenic animals.

In other words, the present invention is aimed to provide an animal individual and an animal cell in which a gene encoding a fatty acid desaturase is introduced thereby the gene is expressed and exert its function, as well as the process for production of the same. In the practice of the present invention, the preferred gene for a fatty acid desaturase may include genes for a fatty acid desaturase that are present in plants but are not present in animals endogenously. More specifically, the preferred gene includes genes for Δ12 and Δ 15 fatty acid desaturases. Preferred genes, however, are not limited to those mentioned above, but may include any other genes or groups of genes encoding fatty acid desaturases that will contribute to the enhanced production of unsaturated fatty acids, provided that they are useful for enhancing the expression of fatty acid desaturases and can function in an animal of interest.

As described above, since animals lack Δ12 and Δ15 fatty acid desaturases, animals can not biosynthesize linoleic acid and α-linolenic acid by themselves, and thus animals must take those fatty acids from a diet. If it were possible to allow a gene for Δ12 or Δ15 fatty acid desaturases derived from plants to be expressed in an animals, it would enable to increase the content of unsaturated fatty acids, which will be beneficial to human health, in the animal. To attain the object of the present invention, either one of a Δ12 or a Δ15 fatty acid desaturase or both may be expressed in animals. By introducing genes for fatty acid desaturases, the contents of linoleic acid and α-linolenic acid can be increased, and that of EPA and DHA can be also increased as well.
As long as Δ12 and Δ15 fatty acid desaturases are functional in an animal, it would be possible for the animal to synthesize all the beneficial fatty acids recognized as such to date at least theoretically.

Use of a gene for a Δ12 fatty acid desaturase derived from roots of spinach is particularly preferred as described in the Examples below. Two types have been known for the Δ12 fatty acid desaturase: one is that present in the membrane of ER (ER membrabe-bound type) while the other is present in the stroma of chloroplast (chloroplast type). In the desaturation reaction of chloroplast type enzyme, a ferredoxin-based electron transport system characteristic for plants is utilized.
On the other hand, the enzyme according to this invention derived from roots of spinach is the ER membrabe-bound type enzyme, and catalyzes the desaturation utilizing an electron transport system of cytochrom b that is also present in animals. Since the present invention is aimed to allow a gene for a fatty acid desaturase to be expressed in an animal, it is preferable to use a gene for the ER membrane-bound type enzyme, which can be easily utilized by animal system.

Genes for fatty acid desaturases suitably used according to the present invention are not necessarily limited to those derived from plants. It had been believed that animals do not have a gene encoding Δ12 or Δ15 fatty acid desaturase. However, recently, Δ12 and Δ15 fatty acid desaturases were isolated from nematode (C. elegans), for the first time from animal species. The fatty acid desaturase derived from such animals may also be used for the present invention, as long as the enzymes are useful to achieve the object of the present invention, that is, increasing the content of unsaturated fatty acids useful for human health. It should be understood that such embodiments are also included within the scope of the present invention.

Host animals suitably used according to the present invention may include any arbitrarily chosen animals, as long as they can be used for the purpose of meat. Livestock for meat included in mammals generally have a low content of unsaturated fatty acids, and are comparatively easy to achieve genetic transformation, and are thus particularly preferred for the purpose of the present invention. Among others, particularly preferred animals to be transformed may include pigs, cattle, goats, sheep and others. Additional suitable animals may include poultry such as chickens, quails, etc., and fish such as tuna, etc.
The range of animals to be transformed according to the invention, however, are not limited to those cited above, but may include a wide variety of animals as long as they can be used as meat for food. In addition to animal individuals transformed to express the gene for fatty acid desaturases, animal cells in which the gene for fatty acid desaturases are introduced is also included in the present invention.

According to the method of the present invention, a transcriptional control region capable of enhancing the expression of a gene for a fatty acid desaturase may be linked upstream of the gene to construct an expression plasmid vector. The genes for fatty acid desaturases may comprise a cDNA or genomic DNA encoding the enzyme. By linking an appropriately chosen transcriptional control region such as a promoter to upstream region of a gene for fatty acid desaturases, it becomes possible to allow the gene to be expressed in various region in a body constitutively or transiently at an arbitrarily time or at a specified time.

Promoters suitably used according to the invention may include, in addition to the chicken β-actin promoter region used in the example below, adipocyte P2 (aP2) promoter, UCP promoter, SV40 promoter, cytomegalovirus promoter, etc. Preferable promoters are not limited, however, to those cited above, but may include various other promoters conventionally used in this technical field. It is possible for a person having an ordinary skill in the art to choose appropriate one from among those promoters depending on the desired part and the desired time at which the gene of interest is to be expressed. If one desires systemic expression of the target gene, the one may preferably select a β-actin promoter. If one desires specific expression of the target gene in adipose tissue, the one may preferably select aP2 promoter, which will control expression of the gene specifically in white and brown adipose tissues.

Plasmids to be used according to the invention are also not limited to any specific ones, but may include various plasmids commonly used in this technical field. The plasmids pUC118 and pBluescript II KS used in the examples below are particularly preferred, but it is also possible to use pSV2, pβA, pZip, pCO12, pME18S, etc.

A vector prepared as above is introduced into early embryos, somatic cells or embryonic stem cells (ES cell) of animals to achieve transformation, and the transformed early embryo, somatic cell or ES cell is allowed to develop until an animal individual. In the examples below, the constructed vector is introduced into an early embryo, but it may also be possible to introduce the vector into animal cells such as somatic cells or ES cells, and to develop animal individuals after the introduction using cloning technique or chimeraplasty. Alternatively, it is also possible to introduce the gene of interest into an arbitrarily or specifically selected tissue or organ of a living body using various techniques to allow the gene to function in the tissue or organ, thereby the gene can be used for biosynthesis of unsaturated fatty acids.
Techniques available for introduction of a vector containing an exogenous gene to be introduced into an early embryo is well known and those having an ordinary skill in the art will be able to modify as appropriate the techniques to perform this invention.

### EXAMPLES

The present invention will be illustrated below by means of examples, but the present invention is not limited in any way to those examples.

### 1. Example 1

### (1) Introduction of the gene for Δ12 fatty acid desaturase into mice and its expression

The experiment was carried out the purpose to investigate whether a plant-derived gene for an ER-membrane bound type fatty acid desaturase, when introduced into an animal, can sufficiently function and convert fatty acids in body fat of the animals into unsaturated fatty acids.

### (2) Preparation of a gene to be introduced

In order to allow the gene for a fatty acid desaturase to be expressed in all of the tissues of a host animal constitutively, a 1.5 kbp fragment containing a chicken β-actin promoter region was ligated upstream of the 1.7 kbp cDNA fragment (HindIII-EcoRI) encoding a gene for a Δ12 fatty acid desaturase (fad2 hereinafter) which had been isolated from roots of spinach and subcloned via pSG5, while an SV40 splicing region and an SV40 poly(A) additional signal region were ligated downstream of the cDNA fragment. The resulting construct was inserted into pUC118 vector. A 4.3 kbp fragment (PstI-BamHI, β-act/fad2) of the constructed plasmid was subjected to 1% agarose gel electrophoresis, purified with Geneclean II (BIO 101, Inc.), and dissolved in TE buffer (pH7.4) to 4 µg/ml. The cloned gene was introduced into mice.

### (3) Production of transgenic mice

Mice (ICR) were purchased from Kiwa Laboratory Animals Co. Handling of these animals was performed in accordance with the "Guidance for Experiment on Animals" (Japan's Society for Animal Experimentation (ed.), Soft Science Publication, 1991). Microinjection of the DNA into a pronuclei of mouse fertilized eggs, and transfer of a live embryo into oviducts of pseudo-pregnant mice were carried out as described by Hogan et al. (Manipulating the Mouse Embryo, A Laboratory Manual, 157-173, Cold Spring Harbor Laboratory Press, New York, 1986). The DNA fragment (β-act/fad2) was microinjected into 1219 fertilized eggs and transferred into recipient animals to obtain 111 pregnant animals. To examine the integration of the transgene into chromosome of each pups, genomic DNA extracted from the tail tissue of the pups was subjected to Southern blotting analysis (Matsumoto et al., Mol. Reprod. Dev., 36, 53-58 (1993)). As a result of the analysis, integration of 1 to 20 copies of the gene were confirmed in the chromosomes of 7 mice (4 females and 3 males).

### (4) Analysis of the mRNA expression of transgene in the transgenic mice

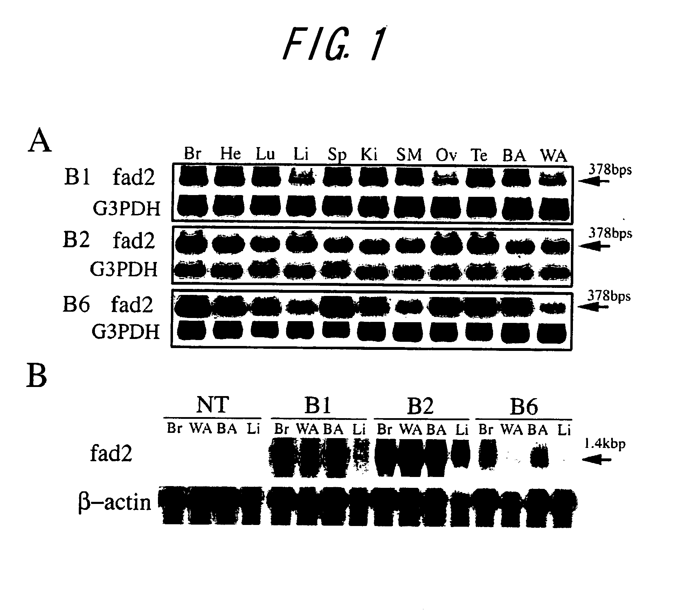

Several lines of the transgenic mice were generated (B1 to B7), and mRNA expression of the transgene of each line was analyzed by RT-PCR and Northern blotting analysis (Fig. 1). In Fig. 1, NT represents the result from wild-type mice, B1 represents B1 transgenic line, B2 represents B2 transgenic line, and B6 represents B6 transgenic line.

Fig. 1A represents the results of RT-PCR analysis performed to evaluate the fad2 mRNA expression levels in the three transgenic lines (B1, B2 and B6). For the RT-PCR analysis, tissues (brain, heart, lung, liver, spleen, kidney, skeletal muscle, ovary, testis, and white and brown adipose tissues) were sampled from the mice respectively, and total RNA was extracted using Trizol (Gibco/BRL). In Fig. 1A, Br represents brain, He represents heart, Lu represents lung, Li represents liver, Sp represents spleen, Ki represents kidney, SM represents skeletal muscle, Ov represents ovary, Te represents testis, BA represents brown adipose tissue, and WA represents white adipose tissue. The arrow indicates the fad2 specific transcriptional product (378 bps). G3PDH indicates the positive control.

Tissues (brain, white adipose tissue, brown adipose tissue and liver) were sampled from mice respectively, and total RNAs were extracted from each tissue using Trizol (Gibco/BRL). Those RNAs were subjected to reverse transcription using AMV reverse transcriptase and random primers (Takara) to provide cDNAs. The cDNAs were amplified by PCR using fad2-specific primers (5'-CTCTCCAATCTACTCGGAC-3' and 5'-ATTGGCTTTATAGCCTTGGT-3'). The amplified products were subjected to 2% agarose gel electrophoresis. As a control, a gene for glyceraldehyde-3-phosphate dehydrogenase (G3PDH) was amplified using primers specific for the gene (Clontech). For the three transgenic lines B1, B2 and B6, the mRNA expression of fad2 was analyzed by RT-PCR. The results are shown in Fig. 1A. Then constitutive expressions of mRNA were observed in all of the tissues of the tested lines (B1, B2 and B6).

To examine whether the full-length mRNA was transcribed from the fad2 gene, Northern blotting analysis was performed.
The results of Northern blotting analysis of fad2 mRNA from the three transgenic lines (B1, B2 and B6) are shown in Fig. 1B. In Fig. 1B, Br represents brain, WA represents white adipose tissue, BA represents brown adipose tissue, and Li represents liver. The arrow indicates fad2-specific transcriptional products (1.4 kbp). β-actin represents a positive control. From B1, B2 and B6 transgenic lines, in which the expression was confirmed by RT-PCR, tissues (brain, white adipose tissue, brown adipose tissue, and liver) of mice were sampled and total RNAs were extracted from the tissues using Trizol.

Poly(A) RNAs were collected from these total RNAs using oligotex-dT (Takara), electrophoresed on 1% agarose-formaldehyde, and blotted onto a nylon membrane (Amersham) in 20xSSC. To the blotted membrane, ³²P-dCTP labeled probe of spinach-derived FAD2 cDNA (1.7 kbp) and probe of mouse s-actin DNA as a control were allowed to hybridize. As a result, 1.4 kb of full-length transcriptional products of FAD2 were detected in all of the mouse tissues examined, and constitutive expression of the gene was confirmed (Fig. 1B). However, the expression levels of the fad2 gene differed among the tissues tested: the expressions in brain and brown adipose tissue were abundant (Fig. 1B). The difference in expression level among different tissues was similar to that described in other reports using chicken s-actin promoter (Matsumoto et al., Mol. Reprod. Dev., 39, 136-140 (1994); Matsumoto et al., Mem. Res. Inst. BOST, Kinki Univ., 2, 11-18 (1999)). This suggests that the transgene for the fatty acid desaturase is transcribed in all tissues in the transgenic mice, and a strong expression was detected in the brown adipose tissue. The expression level in the B1 and the B2 lines were higher than the B6 line. The B1 line was used for further analysis, because of low fertilizing ability of the B2 line.

### (5) Analysis on the protein level expression in the transgenic mice.

To examine the protein expression of the fad2 transgene, total protein was extracted from the brown adipose tissue of the B1 line mice (the mice that showed the strongest level of mRNA expression), and the protein was subjected to Western blotting analysis. The brown adipose tissue was homogenized in sucrose-TM buffer containing protease inhibitor (Complete, Boehlinger Manheim) using Polytron homogenizer. The suspension was centrifuged at 4°C for 10 minutes at 10,000 g.
The supernatant was further centrifuged at 4°C for 60 minutes at 45,000 g, to provide a microsome fraction as the pellet. The pellet was re-suspended in the sucrose-TM buffer and protein concentration of the sample was measured.

An approximately 50 µg of protein was subjected to electrophoresis on 10% SDS polyacrylamide gel and then it was transferred to a PVDF membrane (Millipore). The membrane was incubated for a hour in a blocking solution (Block Ace, Dainippon Pharmaceutical) containing 0.2% Tween 20. The membrane was then washed three times with PBS (pH7.2) containing 0.5% Tween 20, and it was incubated at room temperature for one hour with rabbit anti-FAD2 antiserum (1:2500). The antiserum had been obtained by administering synthetic oligopeptide to a rabbit, an d the oligopeptide corresponded to 18 amino acid residues close to the C-terminal of oleic acid desaturase derived from spinach. The membrane was then washed with PBST, and incubated at room temperature for 30 minutes with an anti-rabbit enzyme-labeled secondary antibody derived from donkey (1:5000, Amersham), and then it washed with TBTS and FAD2 was detected by means of chemiluminescence (ECL, Amersham).

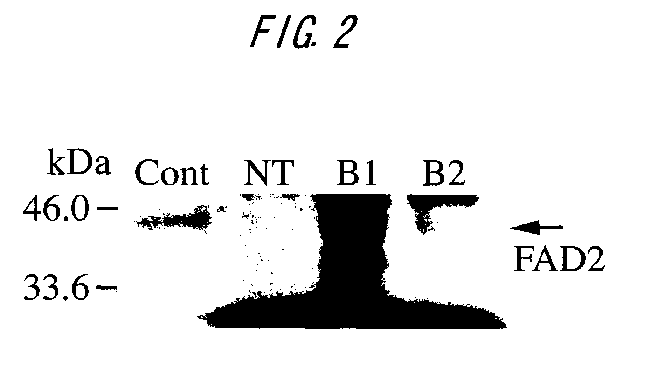

The results are shown in Fig. 2. In Fig. 2, Cont represents the positive control which is the protein extracted from roots of spinach, Nt represents the negative control which is the protein extracted from a wild-type mouse. B1 represents the result from the transgenic mouse of the B1 line, and B2 represents experimental result from the transgenic mouse of the B2 line. The arrow indicates FAD2 (45 kDa). As shown in Fig. 2, a protein indicating molecular weight of about 45 kDa, corresponding to the predicted molecular weight of FAD2 protein derived from spinach, was detected in the microsome fraction obtained from brown adipocytes of the B1 transgenic mouse. This suggests that FAD2 protein in the heterozygous fad2 mice is localized at endoplasmic reticula (ER).

### (6) Alteration in the fatty acid composition in the transgenic mice accompanied with dietary alteration: newly biosynthesis of linoleic acid

Since fatty acid contained in diet is easily incorporated into body fat, it has been known that increased linoleic acid content in a diet leads to increased linoleic acid content in body fat (Hoy and Holmer, Lipids, 25, 455-459 (1990)). In addition, it has been known that fatty acids are easily incorporated into phospholipids mostly (Zevenbergen and Houtsmuller, Biochem. Biophys. Acta, 1002, 312-323 (1989)). Moreover, the linoleic acid content in the brown adipocyte of a mouse fed a normal diet is very high, that is, 24.97% of the total fatty acid content. Therefore, it is expected that the newly synthesized linoleic acid may not be detected because of the presence of linoleic acid derived from the diet. Therefore, the transgenic mice were fed a fat-free diet containing only 5% oleic acid as the source of fat for two weeks. Using these mice, in situ conversion from oleic acid to linoleic acid by spinach-derived FAD2 was investigated. After the feeding, the fatty acid composition in the brown adipose tissue was determined by gas chromatography.

The diet for the mice were as follows: a normal diet (MF, Oriental Yeast, Table 1), a fat-free diet (containing 0.7% fat, Oriental Yeast, Tables 2 and 3), and a high-oleic-acid diet containing 5% oleic acid (01008, Sigma)(Oriental Yeast, Table 4). All of these diets had the same calorie content (340 kCal/100 g).

**TABLE 1:**

| COMPOSITION OF A MOUSE NORMAL DIET | |
|---|---|
| Ingredients | % |
| Coarse protein | 23.8 |
| Coarse fat | 5.1 |
| Coarse fiber | 2.5 |
| Coarse ash | 6.1 |
| Coarse fiber | 3.2 |
| Soluble nitrogen-free substance | 54.0 |

**TABLE 2:**

| COMPOSITION OF A MOUSE FAT-FREE DIET | |
|---|---|
| Ingredients | % |
| Corn starch | 60.0 |
| Casein | 20.0 |
| Sucrose | 10.0 |
| Cellulose powder | 5.0 |
| Mineral mixture | 3.5 |
| Vitamin mixture | 1.0 |
| Methionine | 0.3 |
| Choline bitartrate | 0.2 |

**TABLE 3:**

| COMPOSITION OF A MOUSE FAT-FREE DIET | |
|---|---|
| Ingredients | % |
| Coarse protein | 17.6 |
| Coarse fat | 0.7 |
| Coarse fiber | 8.2 |
| Coarse fiber | 0.4 |
| Coarse fiber | 8.2 |
| Carbohydrate | 63.1 |

**TABLE 4:**

| COMPOSITION OF A MOUSE HIGH-OLEIC-ACID DIET | |
|---|---|
| Ingredients | % |
| Oleic acid | 5.0 |
| Corn starch | 55.0 |
| Casein | 20.0 |
| Sucrose | 10.0 |
| Cellulose powder | 5.0 |
| Mineral mixture | 3.5 |
| Vitamin mixture | 1.0 |
| Methionine | 0.3 |
| Choline bitartrate | 0.2 |

Wild type mice and transgenic mice fed a normal diet were used. The wild-type mice were fed a normal diet, a fat-free diet or a high-oleic-acid diet and the transgenic mice were fed a high-oleic-acid diet for two weeks. After the feeding, the brown adipose tissue was sampled, and the composition of fatty acids was determined. Lipids contained in the brown adipose tissue were extracted in chloroform-methanol (Bligh and Dyer, Can. J. Biochem. Physiol., 37, 911-917 (1959)). The composition of fatty acids in the total lipids was determined by gas chromatography (Wada and Murata, Plant Cell Physiol., 30, 971-978 (1989)).

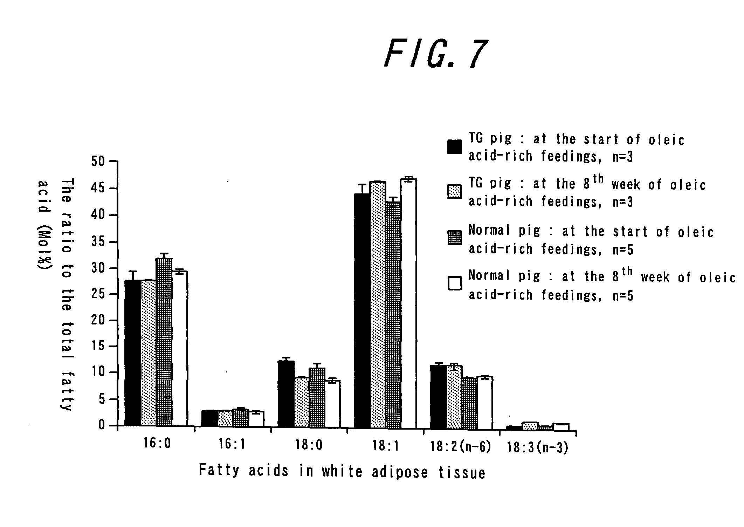

The wild type mice and the B1 line transgenic mice were fed a normal diet, and then fed a fat-free diet or high-oleic-acid diet for two weeks, then the fatty acid composition in the total fat of the brown adipose tissue were determined and shown in Fig. 3. In Fig. 3, each bar represents the mean value, and each error bar represents the standard error. Statistical analysis was performed by variance analysis and then Fischer's PLSD test.

As shown in Fig. 3, the ratio (Mol%) of linoleic acid (18:2n-6) of the total fatty acid content in the B1 trangenic mice fed a high-oleic-acid diet is 1.6 or 1.4 times higher than the corresponding ratios in the wild-type mice fed a fat-free diet or high-oleic-acid diet, respectively (P<0.05). With the increase in the ratio of linoleic acid, the ratio of oleic acid (18:1n-6) decreased to the level of the wild-type mice fed a fat-free diet. The other composition ratios of fatty acids except for linoleic acid of the transgenic mice fed a high-oleic-acid diet were similar to those of the wild-type mice fed a fat-free diet. This suggests that oleic acid contained in the diet is converted to linoleic acid in the B1 transgenic mice. The fact that the ratio of oleic acid that is a substrate for FAD2 decreased and the ration of linoleic acid increased concurrently in the B1 transgenic mice suggests that the transgenic mice has functional enzymatic activity of FAD2.

### 2. Example 2

### (1) Introduction of a gene for Δ12 fatty acid desaturase into pigs and its expression

The experiment was performed for the purpose to investigate whether a plant-derived gene for ER-membrane bound type fatty acid desaturase, when introduced into livestock such as pigs, can convert fatty acids in adipose tissue of the individual into desired polyunsaturated fatty acids (PUFAs). In order to achieve adipose tissue specific expression of the gene for a fatty acid desaturase, the adipocyte P2 (aP2) promoter (Ross et al., Proc. Natl. Acad. Sci. USA, 87, 9590-9594, 1990; Ross et al., Genes & Dev., 7, 1318-1324, 1993), and the gene for Δ12 fatty acid desaturase (fad2) which had been isolated from roots of spinach were ligated to obtain a fused gene (aP2/fad2), and the fused gene was used. In order to achieve efficient conversion of the fatty acid composition by the enzyme, oleic acid (18:1) which serves as a substrate was added to a diet and the pigs were fed the diet for a certain period. This procedure was undertaken based on the finding that the increased content of oleic acid in diet leads to the increased content of oleic acid in adipose tissue (Klingenberg et al., Comp. Biochem. Physiol., 110B, 183-192, 1995). Before feeding a high-oleic-acid diet, and at 8 weeks after feeding a high-oleic-acid diet, the adipose tissue was the collected and the composition of fatty acids was determined.

### (2) Construction of transgene

A transgene was constructed for specific expression of a gene for a fatty acid desaturase in adipocytes of the pigs. A cDNA fragment (1.7 kbp) of the Δ12 fatty acid desaturase gene which had been isolated from roots of spinach was cloned into a vector pBluescript II KS.
An adipocyteP2 (aP2) promoter region (5 kb) that was for specific transgene expression in adipocytes was ligated upstream of the cDNA fragment, and SV40 splicing region and SV40 poly(A) additional signal region were ligated downstream of the same cDNA. The constructed plasmid was cleaved with restriction enzymes SacII and XhoI.
The cleaved DNA fragments were electrophoresed on 1% agarose gel and the DNA fragment of the transgene (about 7.5 kb) was isolated. After purification with Geneclean II (BIO 101, Inc.), the isolated transgene, was dissolved in TE buffer (pH7.4) at the concentration of 4 µg/ml.
The constructs was introduced into pigs.

### (3) Production of transgenic pigs

The fusion gene was microinjected into pronuclei of pig early embryos. Handling of those animals was performed in accordance with the "Guidance for Experiment on Animals" (Japan's Society for Animal Experimentation (ed.), and Soft Science Publication, 1991). Collection of pig embryos, gene injection, and embryo transfer were carried out as follows. Pigs for food of about 13 months old (cross-breeds between Durock (male) and F1 (female) (Landrace x Large White, weighing about 100 kg) received the intramuscular injection of 1000 IU of eCG which was followed by the administration of 500 IU of hCG 72 hours later. Twenty four hours after the administration of hCG, The pigs indicating estrous were mated with male pigs. Twenty-six to thirty hours after the administration of hCG, Stresnil (Azaperon medicine) was administered for tranquilizing followed by inhaled anesthesia. The oviduct was rinsed by upward current through a midventral incision, and embryos were recovered. Immediately thereafter, the fusion gene was microinjected into the pronuclei of the embryos at a concentration of 4 *µ*g/ml.

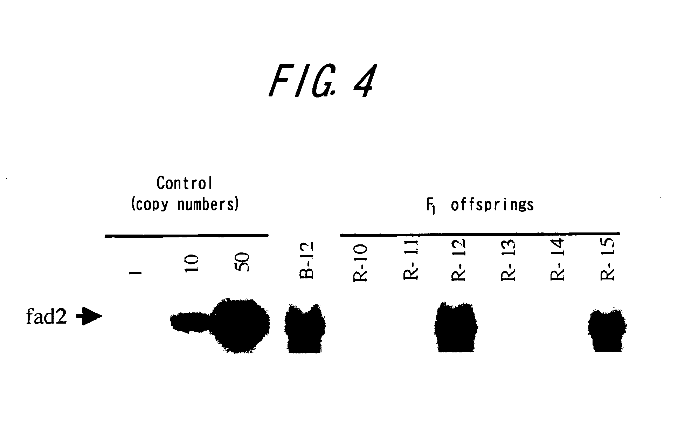

Microinjected embryos with the gene was immediately transferred into the oviducts of female pigs whose estrous cycle had been synchronized with the donor pigs, or of the donor pigs with good ovulation. The recipient females wafter embryo transfer were then housed individually, and allowed to go to term. When 464 pronuclear embryos microinjected with the gene were transferred into 16 recipient pigs synchronized with the donor pigs, and 11 recipients became pregnant and farrowed 70 piglets. Genomic DNA was extracted from the tail tissue of each offspring, and integration of the transgene into a chromosome was examined by Southern blotting analysis. As the result, six transgenic pigs were obtained (Fig. 4).

Integration of the transgene into a chromosome of the transgenic pig was examined by Southern blotting analysis, and the results were shown in Fig. 4. The piglets of the transgenic pig were analyzed with the similar protocol, but the results will be detailed later. In Fig. 4, "control" represents a positive control, and "B-12" represents the result from the transgenic pig (male) developed from an early embryo microinjected with aP2/fad2 gene. "F1 piglets" represents pigs of the next generation produced by mating of the B-12 transgenic pig.
The arrow indicates the bands representing the fad2 gene. In the B-12 that was transgenic, successful integration of the transgene into a chromosome was confirmed. This suggests that the fad2 gene isolated from roots of spinach can be stably integrated into the chromosome of pigs. However, among these six transgenic pigs, two were stillborn, and one was crushed to death after birth.

### (4) Analysis of mRNA expression of the transgene in the transgenic pigs

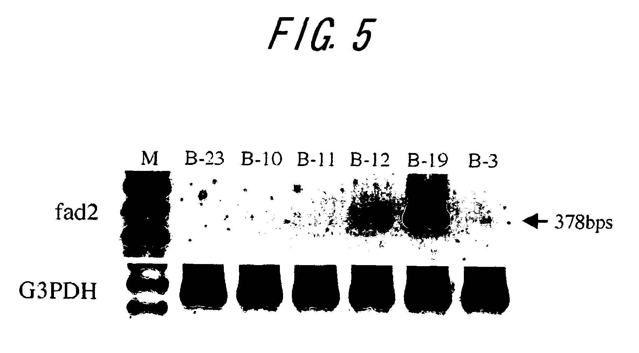

The remaining three pigs survived. At 10 to 11 months of age, they were tranquilized with Azaperon medicine, the dorsal skin slightly caudal from the back of neck was shaved, disinfected thoroughly with isodine/alcohol. A biopsy needling having an internal diameter of 3 mm was inserted through the skin at the depth of about 30 mm from the surface, and then white adipose tissue was biopsied under local anaesthesia with xylocaine. The adipocytes were subjected to RT-PCR and Southern blotting analysis as in Example 1, and the mRNA expression of the trangene was evaluated. Fig. 5 shows the fad2 mRNA expression of white adipose tissue samples from the transgenic pigs (B3, B12 and B19) analysed by RT-PCR method. "M" represents size markers. "B-23," "B-10" and "B-11" represent wild-type pigs, while "B-12," "B-19" and "B-3" represent transgenic pigs. The arrow indicates the fad2 specific transcriptional products (378 bps). From Fig. 5, it was indicated that white adipose tissue of two pigs (B-12 and B-19) out of the three transgenic pigs showed expression of the transgene at mRNA level.

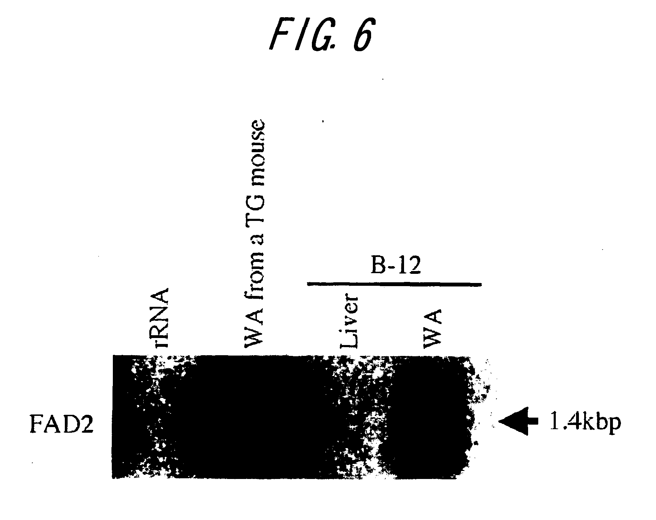

Toexamine whether the full length of fad2 gene was transcribed, Northern blotting analysis was performed. For the B-12 in which expression of the transgene was confirmed by RT-PCR analysis, pigs of the next generation were used, and total RNAs were extracted using Trizol from tissues including white adipose tissue and liver of the pigs. Then, Northern blotting analysis was performed by the similar protocol to Example 1. Fig. 6 shows the fad2 mRNA analysis of the transgenic pig from the B-12 line by Northern blotting analysis. Poly(A) RNAs (2 µg) were subjected to electrophoresis on 1% agarose-formaldehyde, and blotted onto a nylon membrane. The arrow represents the fad2 specific transcriptional products (1.4kbp). "rRNA" represents negative control, "WA from TG mouse" represents the samples of the white adipose tissue of a transgenic mouse in which expression of the transgene is confirmed in Example 1. "Liver" represents the liver, and "WA" represents the white adipose tissue. Fig. 6 indicates that the transgene is transcribed in the adipocytes specifically. This suggests that the fad2 transgene is transcribed specifically in the adipose tissue of the transgenic pig.

### (5) Production of the next generation

The expression of the plant-derived gene for Δ12 fatty acid desaturase was confirmed for the two transgenic pigs (one male and one female), and the inventors produced their offspring for the next generation. This was to examine whether the transgene could be stably transmitted to progenies. In addition to the investigation described above, the transgenic pigs to which the transgene was transmitted stably were suitable to examine whether the newly biosynthesized proteins, resultants of the expression of the transgene, were functional as an enzyme. The female transgenic pig was mated with a wild-type male pig. The female transgenic pig farrowed 12 piglets (three live and nine stillborn). However, all of the three piglets died because of agalactia. The male transgenic pig was mated with two wild-type female pigs.
One female farrowed 14 piglets (nine live and five stillborn), and the other female farrowed 18 piglets (12 live and six stillborn). Genomic DNAs from tail tissues of the 21 live piglets were subjected to Southern blotting analysis. The transgene was transmitted to eight piglets (38%) of the next generation (Fig. 4). In Fig. 4, integration of the transgene into chromosomes was observed in R-12 and R-14, which were both the piglets of the B-12.

### (6) Expression analysis of transgene in transgenic pigs.

It has been reported that the concentration of oleic acid in white adipose tissue of the pigs increased, when pigs are fed a high-oleic-acid diet, (Myer et al., J. Anim. Sci., 70:3734-3741, 1992; Klingenberg et al., Comp. Biochem. Physiol., 110B, 183-192, 1995). In this experiment, transgenic pigs were fed a high-oleic-acid diet, in which 10% oleic acid salad oil (78% oleic acid) was added to the normal pig diet, for 8 weeks. This was to investigate whether oleic acid is converted to linoleic acid in the white adipose tissue of the transgenic pigs by the spinach-derived FAD2. Immediately before the feeding, and 8 weeks after the feeding, the fatty acid composition of the white adipose tissue was determined by gas chromatography by the similar protocol to in Example 1.

Six transgenic pigs (three males and three females) of the B-12 transgenic pig were used for the experiment. They were fed a normal diet (Spurt G, Nosan Corp., Table 5) until five months old. The litters of the transgenic pigs that had no transgene were fed in the same manner as the wild-type pigs, and they were used in the experiment as a control. The feeding conditions were as follows: the male pigs and the female pigs were fed separately, they were fed in concrete-floored feeding rooms of 38 m2, the rooms were lighted for 12 hours daily, the temperature was kept at 20-25°C and five to six pigs were kept in a room. Moreover, the diet and water were available ad libitum. After five months old, they were fed a high-oleic-acid diet (Spurt G supplemented with 10% oleic acid, Nosan Corp., Table 6) for 8 weeks. The B19 line pigs were fed the high-oleic-acid diet similarly as the pigs described above and subjected to experiments because of no offspring from B19. The adipose tissue was frozen immediately after sampling in liquid nitrogen, and kept at - 80°C until analysis of fatty acid composition. Analysis of the fatty acid composition in adipocytes was performed by the similar protocol to Example 1.

**TABLE 5:**

| COMPOSITION OF NORMAL PIG DIET | |
|---|---|
| Ingredients | % |
| Coarse protein | 16.1 |
| Coarse fat | 3.5 |
| Coarse fiber | 2.5 |
| Coarse ash | 4.7 |
| Calcium | 0.74 |
| Phosphor DCP | 0.55 |
| DE | 3420Kcal/Kg |
| TDN | 77.0 |
| DCP | 13 |

**TABLE 6:**

| COMPOSITION OF FATTY ACIDS OF THE DIET GIVEN TO THE TRANSGENIC PIGS | | |
|---|---|---|
| Fatty acid | 10% oleic acid salad oil-supplemented diet (%) | Normal diet (%) |
| Caprylic acid(8:0) | ND | ND |
| Capric acid(10:0) | ND | ND |
| Lauric acid(12:0) | ND | ND |
| Myristic acid(14:0) | 0.02 | ND |
| Myristoleic acid(14:1) | ND | ND |
| Pentadecanoic acid(15:0) | ND | ND |
| Palmitic acid(16:0) | 0.91 | 0.51 |
| Palmitoleic acid(16:1) | 0.03 | 0.02 |
| Heptadecanoic acid(17:0) | ND | ND |
| Stearic acid(18:0) | 0.31 | 0.08 |
| Oleic acid(18:1) | 7.63 | 0.94 |
| Linoleic acid(18:2n-6) | 2.51 | 1.51 |
| Ω-linolenic acid(18:3n-3) | 0.10 | 0.08 |
| Octadecatetraenoic acid(18:4n-3) | ND | ND |
| Arachidonic acid(20:4n-3/n-6) | 0.05 | 0.01 |
| Eicosapentaenoic acid(20:5n-3) | 0.04 | ND |
| ND: Not detected | | |

### (7) Altered composition of fatty acids in transgenic pigs by the alteration of diet―de-novo biosynthesis of linoleic acid

Before the start of the high-oleic-acid feeding (Week 0) and the 8 weeks after feeding pigs with the high-oleic-aciddiet, transgenic pigs and wild-type pigs were tranquilized with Azaperon medicine, the dorsal skin slightly caudal from the back of neck was shave, disinfected thoroughly with isodine/alcohol. A biopsy needling having an internal diameter of 3 mm was inserted through the skin at the depth of about 30 mm from the surface to biopsy white adipose tissue under local anaesthesia with xylocaine. The fatty acid composition in the fat of the adipose tissue was determined as in Example 1.

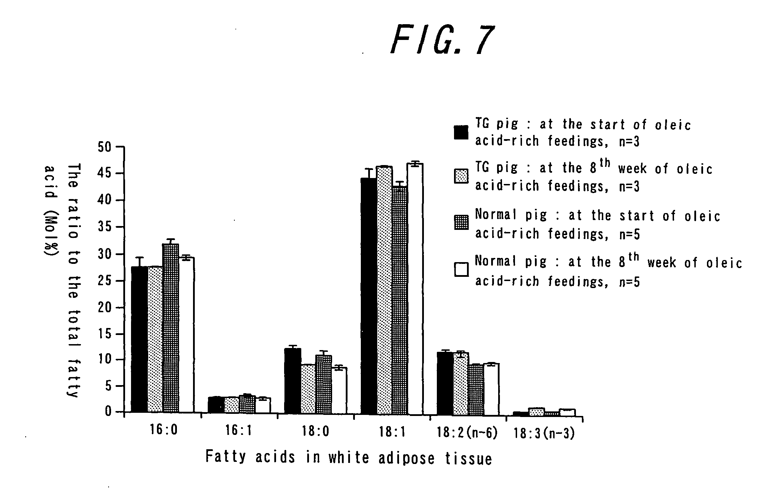

The transgenic pigs carrying aP2/fad2 (the B-12 line) were fed a normal diet for five months, and then fed a high-oleic-acid diet for 8 weeks, then the fatty acid composition in the total fat of the white adipose tissue was eamined. The results were shown in Fig. 7. Each bar represents the mean value, and the error bar represents the standard error. Statistical analysis was performed by variance analysis and then by Fischer's PLSD test. As shown in Fig. 7, the ratio (Mol%) of linoleic acid (18:2n-6) of the total fatty acid content at the start of the high-oleic-acid feeding and at the 8th week of the feeding of the B-12 trangenic pigs are higher than that of the wild-type pigs at 25 and 19%, respectively (P<0.05). The ratio of oleic acid of the wild-type pigs are 5 and 10% higher than that of the transgenic pigs, respectively (P<0.05).

When the ratio of linoleic acid in the white adipose tissue of the B-19 transgenic pigs at the start of the high-oleic-acid feeding was compared with that of wild-type female pigs, the values were 12.9% and 10.3% respectively, and the value of the B-19 transgenic pigs was higher (P<0.05). The ratio of oleic acid was found higher in the transgenic pigs even before the start of the high-oleic-acid feeding. This might be explained as follows: even when fed a normal diet, original level of oleic acid contained in the white adipose tissue of pigs was high, and the FAD2 expressed in the white adipose tissue of the transgenic pig might converted the oleic acid to linoleic acid. However, the ratio of linoleic acid did not increase in the transgenic pigs after fed by the high-oleic-acid feeding. This was probably because linoleic acid was further converted in situ to arachidonic acid to prostanoids. As mentioned above, the fact that the ratio of linoleic acid is increased in the transgenic pigs suggests that FAD2 acts as a functional enzyme in those transgenic pigs.

### 3. Example 3

### (1) Alteration of fatty acid composition in the lipids of in vitro redifferentiated adipocytes derived from the transgenic pig

In order to further investigate on the functional expression of the FAD2 gene in adipocytes of the transgenic pigs, the preadipocytes derived from the transgenic pigs were cultured and differentiated in vitro, then the fatty acid composition of the newly synthesized and accumulated lipids was determined in the differentiated adipocytes. Predipocyte can be prepared as follows. When matured unilocular adipocytes are isolated from mammals, then they were cultured by ceiling culture, accumulated lipid droplets were removed from the cells, and the cells turned to preadipocytes withfibloblast-like shape (Sugihara et al., Differentiation 31, 42-49 (1986)). It was known that, when the cells are subcultured until the number of cells reached to confuluency, then they are induced by the drugs such as insulin, the cells differentiate into adipocytes again and accumulate lipid droplets (Japanese Unexamined Patent Application Publication No. 2000-83656). Since the transgene introduced into a transgenic pig was linked to the aP2 promoter, fad2 did not express in the de-differentiated preadipocytes, however fad2 may express after differentiation. Therefore, the inventors assumed that the function of the fad2 transgene could be investigated in detail by determining the composition of the fatty acids in the accumulated lipid in vitro differentiated preadipocytes derived from the transgenic pigs.

Dorsal adipocytes were biopsied from transgenic pigs or non-transgenic pigs using a biopsy needle in the same manner as in Example 2. The adipose tissue was digested with collagenase solution and the cells were dispersed. The undigested tissue was removed using a filter mesh, then isolated matured unilocular adipocytes were obtained.
The cell suspension was transferred to a culture flask and the flask was filled with 25 mM HEPES buffered Dulbecco's Eagle minimum essential medium (FCS-DMEM) supplemented with 20% fetal bovine serum (FBS). The flask was placed upside-down in an incubator. When cultured in this way, unilocular adipocytes floated on the upper layer of the culture medium and attached to the top surface of the flask.

After incubated for a week, lipid droplets were removed from the adipocytes that were at the top surface of the flask, and became preadipocytes with fibroblast-like shape. The medium filled in the flask was discarded, the flask were turned to original position, the fresh FCS-DMEM was added into the flask, and the preadipocytes were cultured by ordinal cell culture. The cells were subcultured several times, and then incubated for four days in FCS-DMEM supplemented with 5 µg/ml insulin (Wako Pure Chemicals), 0.5 mM isobutylmethyl xanthine (Wako Pure Chemicals) and 0.25 *µ*M dexamethazone (Wako Pure Chemicals) to induce differentiation. The medium was then changed with fresh FCS-DMEM, and the cells were cultured for eight days, thereby lipid droplets were accumulated in the cells. After cell culture, lipids were extracted from the adipocytes, and the fatty acid composition of the lipid was determined as in Example 2 described above.

The results are summarized in Table 7. In the data of Table 7, the analysis of the fatty acid composition was done three times using cell samples of different cell groups. The data with a, b, c and d in Table 7 in the same row with different superscripts indicated significant difference (a, b; P<0.001, c, d; P<0.01). The ratio of linoleic acid of the transgenic adipocytes was 27.7%, and the value was much higher than that of the wild-type cells (7.2%, P<0.001). In the adipocytes of transgenic cells, the ratio of palmitic acid (16:0) was 16.3%, and the value was much lower and about half value compared with that of wild-type cells (34.2%, P<0.001). Since mammalian cells have the activity of Δ9 fatty acid desaturase, animal cells contain large amount of oleic acid. Presumably, oleic acid converted to linoleic acid by functional expression of the Δ12 fatty acid desaturase in the adipocytes of transgenic pigs, consequently the ratio of linoleic acid was increased greatly. Accompanied with the increase in the linoleic acid ratio, it is assumed that the palmitic acid ratio is decreased in order to compensate the oleic acid. In the adipose tissue of the transgenic pigs, decrease in the palmitic acid ratio and increase in the linoleic acid ratio were also observed (Fig. 7). However, the fatty acid composition can be determined in lipids accumulated in adipocytes by in vitro culture. Therefore, great difference in the fatty acid composition could be observed in the cells of the transgenic pigs. These results indicate that FAD2 cDNA in the transgenic pigs is expressed functionally in the adipocytes and the biosynthesis of linoleic acid.

**TABLE 7:**

| THE COMPOSITION OF FATTY ACIDS IN RE-DIFFERENTIATED PREADIPOCYTES DERIVED FROM TRANSGENIC PIGS BY IN VITRO CULTURE | | |
|---|---|---|
| Fatty acids | Mol% | |
| | Wild type pig | Transgenic pig |
| 16:0 | 34.2±1.5a | 16.3±0.7b |
| 16:1 | 0.1±0.1 | 0.03±0.03 |
| 18 : 0 | 9.6±0.9 | 14.2±1.8 |
| 18: 1 | 47.2±2.4 | 39.1±2.8 |
| 18: 2n-6 | 7.3±0.6a | 27.7±1.5b |
| 18: 3n-3 | 0.3±0.1 | 0.2±003 |
| 20:0 | 0.2±0.1 | 0.1±0.03 |
| 20: 4n-6 | 0.8±0.05C | 1.9±0.2d |
| 22 : 5n-3 | 0.3±0.1 | 0.5±0.1 |

For analysis of the composition of fatty acids, analysis of the fatty acid composition was done three times using cell samples of different cell groups. The values in the same row with different superscripts indicated significant difference (a, b; P<0.001, c, d; P<0.01).

The present invention provides transgenic animals, in which enhanced level of unsaturated fatty acids that are beneficial to human health, by introduction of a gene for a fatty acid desaturase into the animals, and a method for enhancement of contents of unsaturated fatty acids in animals. The transgenic animals and the method for production of the transgenic animals in this invention are particularly useful for the production of meat beneficial to human health.

## Claims

1. A transgenic animal in which a gene for a fatty acid desaturase is introduced into the animal to transform the animal to allow the gene for a fatty acid desaturase to be expressed in the animal, thereby the content of unsaturated fatty acid is increased in the animal.

2. The transgenic animal according to Claim 1 wherein the gene for a fatty acid desaturase is (a) genes for a Δ12 fatty acid desaturase and/or a gene for a Δ15 fatty acid desaturase.

3. The transgenic animal according to Claim 2 wherein the gene for a fatty acid desaturase is a gene for Δ12 fatty acid desaturase derived from the root of spinach.

4. The transgenic animal according to Claim 1 wherein the gene for a fatty acid desaturase is a gene for a Δ12 fatty acid desaturase derived from roots of spinach, and expression of the gene is promoted by a chicken β-actin promoter or by an adipocyte P2 (aP2) promoter.

5. The transgenic animal according to Claim 1 wherein the unsaturated fatty acid is linoleic acid, α-linolenic acid, eicosapentaenoic acid (EPA) or docosahexaenoic acid (DHA).

6. The transgenic animal according to Claim 1 wherein the animal is for a source of meat.

7. The transgenic animal according to Claim 6 wherein the animal is a mammal.

8. The transgenic animal according to Claim 7 wherein the mammal is a pig, cattle, goat or sheep.

9. A transgenic animal cell in which a gene for a fatty acid desaturase is introduced into the animal cell to transform the animal cell to allow the gene for a fatty acid desaturase to be expressed in the animal cell, thereby the content of unsaturated fatty acid is increased in the animal cell.

10. A method for increasing the content of unsaturated fatty acids in an animal, the method comprising the steps of introducing a gene for fatty acid desaturase in an animal, and transforming the animal to allow the gene for fatty acid desaturase to be expressed in the animal.

11. A method for increasing the content of unsaturated fatty acids in an animal, the method comprising the steps of constructing a vector containing a gene for a fatty acid desaturase with a transcriptional control region that enables expression of the gene for fatty acid desaturase, transforming animals by introduction of the vector into early embryos, somatic cells or embryonic stem cells (ES cells) of the animals, and allowing the gene for a fatty acid desaturase to be expressed in the animals that were developed from the transformed early embryos, somatic cells or embryonic stem cells.

12. The method according to Claim 10 wherein the gene for a fatty acid desaturase is a gene for a Δ12 fatty acid desaturase and/or a gene for a Δ15 fatty acid desaturase.

13. The method according to Claim 12 wherein the gene for a fatty acid desaturase is a gene for a Δ12 fatty acid desaturase derived from roots of spinach.

14. The method according to Claim 11 wherein the gene for a fatty acid desaturase is a gene for Δ12 fatty acid desaturase derived from the root of spinach, and the transcriptional control region is a chicken β-actin promoter or an adipocyte P2 (aP2) promoter.

15. The method according to Claim 10 wherein the unsaturated fatty acid is linoleic acid, α-linolenic acid, eicosapentaenoic acid (EPA) or docosahexaenoic acid (DHA).
